# EUROPEAN PATENT APPLICATION

(11) **EP 3 808 756 A1**
(43) Date of publication of application: **21.04.2021**
(21) Application number: 20193953.5
(22) Date of filing: 17.07.2015
(51) Int. Cl.: C07G 15/00, C07K 14/47, C12Q 1/68, G01N 33/543, G01N 33/569, C07K 14/74

(54) **METHODS FOR USING EXOSOMES TO MONITOR TRANSPLANTED ORGAN STATUS**

(30) Priority: 17.07.2014 US 201462025858 P
(62) Divisional of application: 15822761.1
(71) Applicant: The Trustees of The University of Pennsylvania, Philadelphia, PA 19104 (US)
(72) Inventor: VALLABHJOSYULA, Prashanth, Bryn Mawr, PA 19010 (US); TAYLOR, Douglas, Monmouth Junction, NJ 08852 (US)
(74) Representative: Barker Brettell LLP

(57) **Abstract**

This present disclosure relates to the use of donor organ-derived microvesicles to monitor the status of a transplanted organ in a subject. Accordingly, this disclosure provides for methods and kits for isolating, purifying and/or identifying donor organ-derived microvesicles from a biological sample of a subject. In certain embodiments, a method for isolating, purifying and/or identifying donor organ-derived microvesicles includes obtaining a biological sample from the subject and isolating, purifying or identifying a donor organ-derived microvesicle from the biological sample by the detection of a protein specific for the donor.

## Description

### CROSS-REFERENCE TO RELATED APPLICATIONS

This application claims priority to U.S. Provisional Patent Application Serial No. 62/025,858, filed July 17, 2014, the contents of which are incorporated by reference herein in their entirety.

### BACKGROUND

Transplantation of a graft organ or tissue from a donor to a host patient is a feature of certain medical procedures and treatment protocols, and can significantly improve patient survival and quality of life. Despite efforts to avoid graft rejection through host-donor tissue type matching, in transplantation procedures, where a donor organ is introduced into a host, immunosuppressive therapy is generally required to the maintain viability of the donor organ in the recipient. However, despite the wide use of immunosuppressive therapy, many patients suffer from complications associated with the transplanted organ, such as acute and chronic rejection, over-immunosuppression and infection.

Early detection of organ rejection is a clinical concern in the care of transplant recipients. Detection of rejection before the onset of graft dysfunction allows successful treatment of this condition with aggressive immunosuppression. In addition, it is equally important to reduce immunosuppression in patients who do not have organ rejection to minimize drug toxicity. Many kidney transplant rejection episodes are detected by periodically measuring the function of the transplanted kidney, for example by using biochemical tests such as assays that measure serum creatinine concentrations. Additionally, in liver transplantation, monitoring of liver enzymes in the serum of the recipient is used to monitor the status of the transplanted liver.

Small microvesicles released by cells are known as exosomes (Thery et al., 2002). Exosomes are tissue and major histocompatibility complex (MHC) specific microvesicles (30-200 nm) with stable RNA cargo reflecting the conditional state of the tissue releasing them. Exosomes also take part in the communication between cells by functioning as transport vehicles for proteins, RNAs, DNAs, viruses and prions. Since their discovery, a growing number of therapeutic applications are in development using exosomes derived from various producing cells, such as dendritic cells (DC), T lymphocytes, tumor cells and cell lines (Thery et al., 2002 and Delcayre et al., 2005). For instance, DC-derived exosomes (also designated dexosomes) pulsed with peptides derived from tumor antigens elicit anti-tumor responses in an animal model for the matching tumor (Zitvogel et al., 1998). Two Phase-I clinical trials using autologous dexosomes for the treatment of lung cancer (Morse et al., 2005) and melanoma (Escudier et al., 2005), respectively, have recently been completed. There exists a critical need for a method for monitoring transplanted organ status.

### SUMMARY

The present disclosure provides methods for monitoring the status of a transplanted organ in a subject. The present disclosure further provides methods for isolating, purifying and/or detecting donor-derived microvesicles from a biological fluid of a transplant recipient.

In certain embodiments, a method for monitoring the status of a transplanted organ in a subject can include (a) obtaining a biological sample from the subject; and (b) isolating, purifying and/or identifying one or more donor organ-derived microvesicles from the biological sample. In certain embodiments, the method can further include the isolation of tissue-specific microvesicles.

In certain embodiments, a method for the isolation, identification and/or purification of donor organ-derived microvesicles can include (a) obtaining a biological sample from the subject; and (b) isolating, purifying and/or identifying one or more donor organ-derived microvesicles from the biological sample by the detection of a marker specific for the donor. In certain embodiments, the marker can be a protein present on the surface of the microvesicles. In certain embodiments, the protein can be a member of the major histocompatibility complex (MHC) that is specifically present on the surface of the donor organ-derived microvesicles.

In certain embodiments, a method for the isolation, identification and/or purification of donor organ-derived microvesicles can include (a) obtaining a biological sample from the subject; and (b) isolating, purifying and/or identifying one or more donor organ-derived microvesicles from the biological sample by the detection of a marker specific for the organ that was transplanted in the recipient. For example, and not by way of limitation, the protein can be a cell surface protein that is specific to the cell type of the donor organ.

In certain non-limiting embodiments, the biological sample can be a bodily fluid such as blood, urine, saliva, nasotracheal secretions, amniotic fluid, breast milk or ascites. In certain embodiments, the biological sample can be a blood sample. In specific non-limiting embodiments, the microvesicles can be purified, isolated and/or obtained in one or more biological samples from a subject. The use of a bodily fluid as a biological sample makes it possible to eliminate invasiveness of the diagnostic or prognostic procedure, and dramatically reduces the burden of the examination on the subj ect.

The disclosure also provides kits for monitoring the status of a transplanted organ of a subject, where the kit containing reagents useful for isolating, purifying and/or identifying the donor organ-derived microvesicles in a biological sample.

### BRIEF DESCRIPTION OF THE DRAWINGS

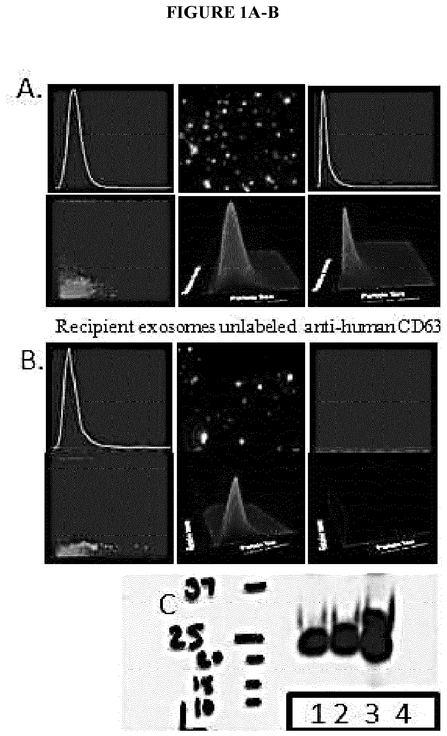

**Figure 1A-C****. Xenoislet transplantation.** (A) Nanosight in light scatter and fluorescent mode for xenotransplant. (B) Nanosight for negative control. (C) Western blot of microvesicles with anti-human CD63 from 3 xenotransplants (lanes 1-3) and negative control (lane 4).

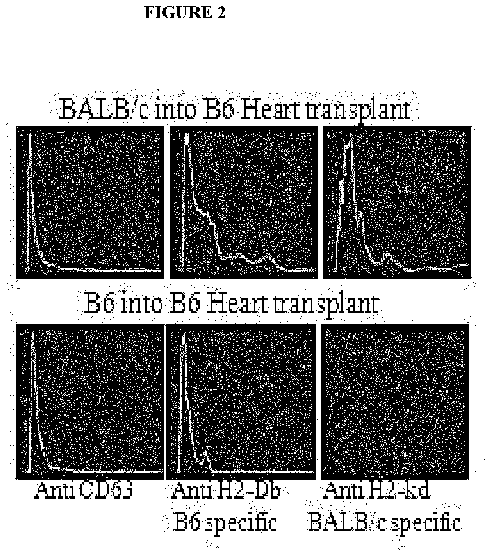

**Figure 2****.** Nanosight analysis on fluorescent mode for BALB/c (donor) and B6 (recipient) specific antibody quantum dot analysis is shown for full mismatch and negative control.

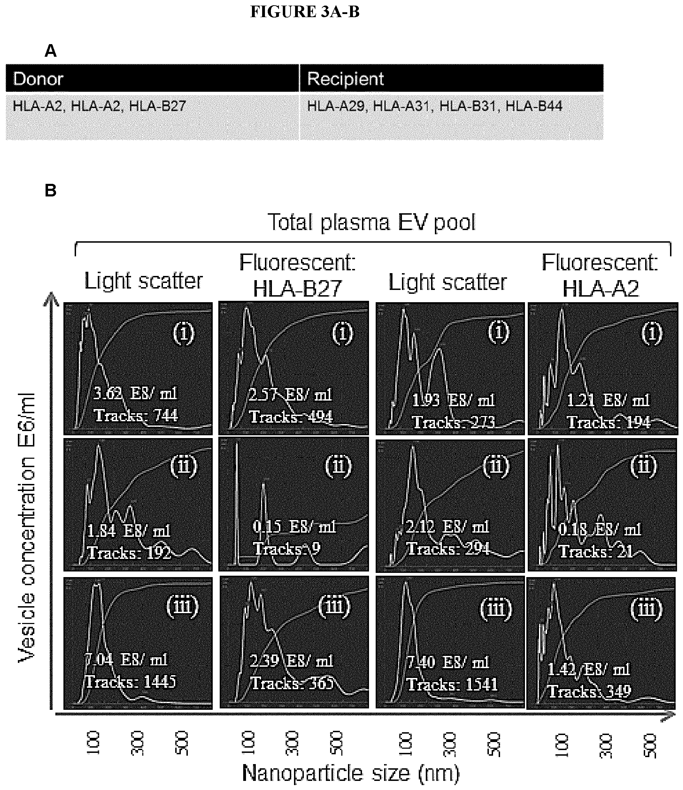

**Figure 3A-E****. Detection and purification of exosomes from human renal transplant recipients.** (A) Relevant donor-recipient human leukocyte antigen (HLA) profiles are shown. (B) On Nanosight, HLA-A2 and HLA-B27 positive exosomes were seen in the donor plasma (i), and recipient plasma post-transplant day 4 (iii), but not in the recipient plasma pre-transplant (ii). (C) On Western blot, HLA-A2 bound plasma exosomes fractions from recipient pre-transplant and post-kidney implantation but pre-perfusion of the donor organ were negative for aquaporin 2 (renal epithelial marker), but the recipient post-transplant day 4 sample was positive for aquaporin 2. (D) Urinary exosomes were analyzed for HLA-A2 presence. (E) HLA-A2 bound urinary exosomes fractions from postoperative day 4 and day 30 showed expression of renal glomerular marker, podocalyxin-1, but such expression was not observed in the pre-transplant sample.

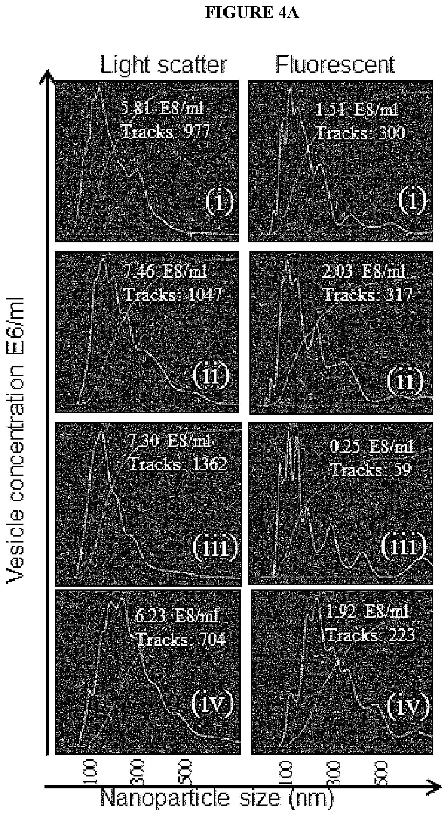

**Figure 4A-D****. Detection of transplant human islet signal in recipient mouse plasma exosomes in a xenoislet transplant model.** (A) Exosomes were analyzed on the NanoSight NS300 fluorescence mode using anti-HLA-A quantum dot. HLA-A positive signal was detected in xenoislet exosomes (i), human plasma exosomes (positive control) (ii), but not in naive mouse (iii) plasma exosomes (p<0.0001). Exosomes isolated from *in vitro* human islet culture supernatant also stained positive for HLA-A signal (iv). (B) Similar results were obtained when exosomes were labeled with another human specific MHC, HLA-C quantum dot. (C) Western blot analysis confirmed HLA-A and HLA-C expression in total plasma exosome pool of xenoislet mouse sample, *in vitro* islet culture supernatant exosomes, human plasma exosomes, but not in naive mouse plasma exosomes. (D) Islet graftectomy was performed in 6 xenoislet animals. In plasma exosome sample from day 21 after islet graftectomy, the HLA-A quantum dot signal was undetectable on NanoSight.

### DETAILED DESCRIPTION

The present disclosure provides techniques related to the use of microvesicles, identified herein, to monitor the status of a transplanted organ in a subject. The present disclosure, in certain embodiments, further provides methods for isolating, purifying and/or identifying donor organ-derived microvesicles released into the bodily fluids of a subject. In certain embodiments, the present disclosure provides for methods and kits for isolating, purifying and/or identifying one or more donor organ-derived microvesicles in a biological sample of a subject.

Medical practices rely on histological or clinical parameters to monitor the status and/or function of transplanted hearts and lungs, and there are no existing methods of non-invasively detecting monitoring transplanted hearts or lungs. For example, for patients undergoing cardiac transplantation, surveillance endomyocardial biopsies are taken at weekly intervals to 6 weeks, then at 2 weekly intervals until 3 months. In addition, any positive biopsy is followed-up by a repeat biopsy one week later to ensure that anti-rejection therapy has been successful. Patients also undergo further biopsies when clinically indicated, resulting in a patient undergoing multiple biopsies within the first year of transplantation. Therefore, detection of donor organ-derived microvesicles circulating in a biological fluid of a recipient, as described herein, provides for a non-invasive and time-sensitive assay to monitor the status of a transplanted organ, especially, in the case of, transplanted heart and lungs.

### Definitions

As used herein, "transplantation" refers to the process of taking a cell, tissue or organ, called a "transplant" or "graft" from one subject and placing it or them into a (usually) different subject. The subject who provides the transplant is called the "donor" and the subject who received the transplant is called the "recipient." An organ, or graft, transplanted between two genetically different subjects of the same species is called an "allograft." A graft transplanted between subjects of different species is called a "xenograft." Examples of transplanted organs that can be monitored by the methods disclosed herein include, but are not limited to, heart, lungs, kidney, liver, islets and pancreas.

As used herein, the term "biological sample" refers to a sample of biological material obtained from a subject, *e.g.,* a human subject, including a biological fluid, *e.g.,* blood, plasma, serum, urine, sputum, spinal fluid, pleural fluid, nipple aspirates, lymph fluid, fluid of the respiratory, intestinal, and genitourinary tracts, tear fluid, saliva, breast milk, fluid from the lymphatic system, semen, cerebrospinal fluid, intra-organ system fluid, ascitic fluid, tumor cyst fluid, amniotic fluid, bronchoalveolar fluid, biliary fluid and combinations thereof. In certain non-limiting embodiments, the donor organ-derived microvesicles are isolated and/or purified from a blood sample obtained from a subject. In certain non-limiting embodiments, the donor organ-derived microvesicles are isolated and/or from a urine sample obtained from a subj ect.

The term "patient" or "subject," as used interchangeably herein, refers to any warm-blooded animal, *e.g*., a human. Non-limiting examples of non-human subjects include non-human primates, dogs, cats, mice, rats, guinea pigs, rabbits, fowl, pigs, horses, cows, goats, sheep, etc.

The term "microvesicle" as used herein, refers to vesicles that are released from a cell. In certain embodiments, the microvesicle is a vesicle that is released from a cell by exocytosis of intracellular multivesicular bodies. In certain embodiments, the microvesicles can be exosomes.

As used herein, the term "transplant rejection," is defined as functional and/or structural deterioration of an organ. Transplant rejection can include functional and/or structural deterioration due to an active immune response expressed by the recipient, and independent of non-immunologic causes of organ dysfunction. Transplant rejection can include donor organ injury, such as an infection of the transplant organ.

### Methods

The present disclosure, in certain embodiments, provides methods for monitoring the conditional state of a transplanted organ by monitoring the microvesicles released from the donor organ. In certain embodiments, the present disclosure provides methods for isolating, detecting and/or purifying microvesicles derived from a donor organ transplanted in a recipient for monitoring the conditional state of a transplanted organ. In certain embodiments, the methods of the present disclosure can further include the detection of one or more markers specific to the donor organ to confirm the isolation and/or purification of donor organ-derived microvesicles, *e*.*g*., exosomes.

In certain embodiments, the methods for monitoring the conditional state of a transplanted organ in a subject include: (a) obtaining a biological sample from the subject; and (b) isolating, purifying and/or identifying one or more donor organ-derived microvesicles from the biological sample. In certain embodiments, the method can include enriching donor organ-derived microvesicles for microvesicles originating from a specific cell type and/or tissue. In certain embodiments, the method can include confirming that the donor organ-derived microvesicles originated from a specific cell type and/or tissue.

In certain embodiments, a method for the isolation, identification and/or purification of donor-derived microvesicles can include: (a) obtaining a biological sample from the subject; and (b) isolating, purifying and/or identifying one or more donor organ-derived microvesicles from the biological sample by the detection of a marker specific for the donor. In certain embodiments, the marker can be a protein. For example, and not by way of limitation, the protein can be a member of the major histocompatibility complex (MHC) classes of proteins that is specific to the donor, as detailed below.

In certain embodiments, a method for the isolation, identification and/or purification of donor-derived microvesicles can include: (a) obtaining a biological sample from the subject; and (b) isolating, purifying and/or identifying one or more donor organ-derived microvesicles from the biological sample by the detection of a marker specific for the organ that was transplanted in the recipient. For example, and not by way of limitation, the marker can be a microvesicle surface protein that is specific to the cells of the donor organ, *e.g.,* a protein specific to a kidney cell, as detailed below.

In certain embodiments, a method for the isolation, identification and/or purification of donor-derived microvesicles can include (a) obtaining a biological sample from the subject; (b) isolating and/or purifying one or more microvesicles from the biological sample to generate a microvesicle sample; and (c) isolating, purifying and/or identifying one or more donor organ-derived microvesicles from the microvesicle sample by the detection of a marker specific for the organ that was transplanted in the recipient and/or by the detection of a marker specific for the donor. In certain embodiments, the microvesicle sample can include microvesicles derived from the donated organ (or cells thereof) and/or microvesicles derived from a recipient organ (or cells thereof).

In certain embodiments, the donor organ-derived microvesicles can be exosomes. In certain embodiments, the microvesicles can be in the size range from about 30 nm to 1000 nm. For example, and not by way of limitation, the microvesicles can be from about 30 nm to about 900 nm, from about 30 nm to about 800 nm, from about 30 nm to about 700 nm, from about 30 nm to about 600 nm, from about 30 nm to about 500 nm, from about 30 nm to about 400 nm, from about 30 nm to about 300 nm, from about 30 nm to about 200 nm, from about 30 nm to about 100 nm or from about 30 nm to about 50 nm in size.

In certain embodiments, and as noted above, methods for assessing the conditional state of a transplanted organ in the subject and/or the isolation and/or purification of donor-derived microvesicles from a subject include obtaining at least one biological sample from the subject. In certain embodiments, the microvesicles can be detected in blood (including plasma or serum) or in urine, or alternatively at least one microvesicle can detected in one sample, *e.g*., the blood, plasma or serum, and at least one other microvesicle is detected in another sample, *e.g*., in urine. The step of collecting a biological sample can be carried out either directly or indirectly by any suitable technique. For example, and not by way of limitation, a blood sample from a subject can be carried out by phlebotomy or any other suitable technique, with the blood sample processed further to provide a serum sample or other suitable blood fraction.

In certain embodiments, the information provided by the methods described herein can be used by the physician in determining the most effective course of treatment (*e*.*g*., preventative or therapeutic). A course of treatment refers to the measures taken for a patient after the assessment of increased risk for organ rejection is made. For example, when a subject is identified to have an increased risk of organ rejection, the physician can determine whether frequent monitoring of donor organ-derived microvesicles is required as a prophylactic measure.

### Microvesicle Isolation Techniques

Circulating donor-organ derived microvesicles can be isolated from a subject by any means known in the art and currently available. Circulating donor-organ derived microvesicles can be isolated from a biological sample obtained from a subject, such as a blood sample, or other biological fluid. In certain embodiments, the circulating donor-organ derived microvesicles can be isolated from the urine of the recipient. In certain embodiments, the microvesicles can be exosomes.

There are several capture and enrichment platforms that are known in the art and currently available. For example, microvesicles can be isolated by a method of differential centrifugation as described by Raposo et al., 1996. Additional methods include anion exchange and/or gel permeation chromatography as described in U.S. Pat. Nos. 6,899,863 and 6,812,023. Methods of sucrose density gradients or Organelle electrophoresis are described in U.S. Pat. No. 7,198,923. A method of magnetic activated cell sorting (MACS) is described in Taylor and Gercel-Taylor, 2008. A method of nanomembrane ultrafiltration concentrator is described in Cheruvanky et al., 2007. Microvesicles can be identified and isolated from a biological sample of a subject by a newly developed microchip technology that uses a unique microfluidic platform to efficiently and selectively separate microvesicles (Nagrath et al., 2007). This technology can be adapted to identify and separate microvesicles using similar principles of capture and separation.

In certain embodiments, the donor organ-derived microvesicles can be purified, isolated and/or identified by the detection of a marker specific to the donor organ and/or to the cell type of the donated organ. In certain embodiments, the marker can be nucleic acids and/or proteins that reside on the surface or within the microvesicles. The microvesicles, *e*.*g*., exosomes, can be isolated from a biological sample and analyzed using any method known in the art. The nucleic acid sequences, fragments thereof, and proteins, and fragments thereof, can be isolated and/or identified in a biological sample using any method known in the art, as described below.

In certain embodiments, the microvesicles isolated from a biological sample can be enriched for those released from the donor organ through the use of the MHC (Major histocompatibility complex) proteins that reside on the surface of the microvesicles. For example, and not by way of limitation, the donor organ-derived microvesicles can be isolated by the detection of MHC proteins specific to the donor microvesicles as compared to the microvesicles released by the recipient, *e*.*g*., by the detection of a specific allele of *HLA-A* and/or *HLA-B* genes as compared to the recipient. In certain embodiments, the methods of the present disclosure can include identifying the specific MHC proteins present on the surface of donor-derived microvesicles as compared to recipient organ-derived microvesicles, followed by the isolation of donor-derived microvesicles by the detection of an identified specific MHC protein. For example, and not by way of limitation, donor organ-derived microvesicles can be isolated through the use of beads, *e*.*g*., magnetic beads, that are conjugated to antibodies that are specific for a protein present on the surface of donor organ-specific microvesicles.

In certain non-limiting embodiments, high exclusion limit agarose-based gel chromatography can be utilized to isolate microvesicles from a recipient's plasma (Taylor et al., 2005). For example, and not by way of limitation, to isolate the total vesicle fraction, the plasma sample can be fractionated using a 2.5 x 30cm Sepharose 2B column, run isocratically with PBS, and the elution can be monitored by absorbance at 280nm. The fractions comprising microvesicles can be concentrated to 2ml using an Amicon ultrafiltration stirred cell with a 500K Dalton cut-off membrane and can used for the affinity separation of organ-specific microvesicles subpopulations. Since microvesicles within the circulation are generated from multiple cell types, affinity based approaches can be used to specifically purify subsets of microvesicles (Taylor et al., 2005). For immunosorbent isolation of transplant organ derived microvesicle populations, recipient plasma microvesicles can be selectively incubated with antibodies specific for the donor's MHC profile coupled with magnetic microbeads. After incubation for 2 hours at 4°C, the magnetic bead complexes can be placed in the separator's magnetic field and the unbound microvesicles can be removed with the supernatant. The bound donor-derived microvesicle subsets can be recovered and diluted in IgG elution buffer (Pierce Chemical Co), centrifuged and resuspended in PBS. Donor microvesicle concentration and size distribution can be determined using the NanoSight NS300. Additional methods to isolate microvesicles include, but are not limited to, ultracentrifugation and sucrose gradient-based ultracentrifugation. In certain embodiments, the microvesicle isolation kit, EXOQUICK™, and/or the EXO-FLOW™ system from System Bioscience, Inc. can be used.

In certain embodiments, the microvesicles isolated from a biological sample can be enriched for those originating from a specific cell type, for example, but not limited to, lung, pancreas, stomach, intestine, bladder, kidney, ovary, testis, skin, colorectal, breast, prostate, brain, esophagus, liver, placenta or fetus cells. In addition, the microvesicles often carry surface molecules such as antigens from their donor cells, surface molecules, *e*.*g*., proteins, can be used to identify, isolate and/or enrich for microvesicles from a specific cell type (Al-Nedawi et al., 2008; Taylor and Gercel-Taylor, 2008). In certain embodiments, the microvesicles can be isolated from a biological sample of a subject and enriched for those originating from the cell of the organ that was transplanted into the subject.

In certain embodiments, microvesicles can be isolated based on the proteins residing on the surface of the microvesicles, which are specific to the cell type from which it originated. For example, and not by way of limitation, the surface antigen can be epithelial-cell-adhesion-molecule (EpCAM), which is specific to microvesicles from cells of lung, colorectal, breast, prostate, head and neck, and hepatic origin, but not of hematological cell origin can be used to isolate microvesicles (Balzar et al., 1999; Went et al., 2004). In certain embodiments, the surface antigen can be CD24, which is a glycoprotein specific to urine microvesicles, and can be used to isolate and/or purify microvesicles from the kidney (Keller et al., 2007). In certain embodiments, the surface antigen can be the renal glomerular marker, Podocalyxin-1, which can be used to isolate and/or purify microvesicles from the kidney. In certain embodiments, the surface antigen can be the renal epithelial marker, Aquaporin 2, which can be used to isolate and/or purify microvesicles from the kidney.

The isolation of microvesicles from specific cell types and/or donor organs can be accomplished, for example, by using antibodies, aptamers, aptamer analogs or molecularly imprinted polymers specific for a desired surface antigen. In certain embodiments, the surface antigen is specific for a cell type of a specific organ. One non-limiting example of a method of microvesicle separation based on cell surface antigen is provided in U.S. Pat. No. 7,198,923. As described in, *e.g*., U.S. Pat. Nos. 5,840,867 and 5,582,981, WO 2003/050290 and a publication by Johnson et al., 2008, aptamers and their analogs specifically bind surface molecules and can be used as a separation tool for retrieving cell type-specific microvesicles. Molecularly imprinted polymers also specifically recognize surface molecules as described in, *e.g*., U.S. Pat. Nos. 6,525,154, 7,332,553 and 7,384,589 and Bossi et al., 2007 and are a tool for retrieving and isolating cell type-specific microvesicles. In certain embodiments, microvesicles can be isolated based on the MHC complex residing on the surface of the microvesicles.

### Protein Detection Techniques

In certain embodiments, the methods of the present disclosure can include the detection of one or more markers specific to the donor and/or the donor organ to confirm the proper isolation and/or purification of donor organ-derived exosomes. In certain embodiments, the marker for donor organ-derived exosomes can be a protein present on the surface and/or within the donor organ-specific isolated microvesicles, *e*.*g*., exosomes. Proteins can be isolated from a microvesicle using any number of methods, which are well-known in the art, the particular isolation procedure chosen being appropriate for the particular biological sample. In certain embodiments, the protein can be detected on the surface of the microvesicle.

Methods for the detection of proteins are well known to those skilled in the art, and include but are not limited to mass spectrometry techniques, 1-D or 2-D gel-based analysis systems, chromatography, enzyme linked immunosorbent assays (ELISAs), radioimmunoassays (RIA), enzyme immunoassays (EIA), Western Blotting, immunoprecipitation and immunohistochemistry. These methods use antibodies, or antibody equivalents, to detect protein. Antibody arrays or protein chips can also be employed, *see* for example U.S. Patent Application Nos: 2003/0013208A1; 2002/0155493A1, 2003/0017515 and U.S. Pat. Nos. 6,329,209 and 6,365,418, herein incorporated by reference in their entirety.

ELISA and RIA procedures can be conducted such that a protein standard is labeled (with a radioisotope such as ¹²⁵I or ³⁵S, or an assayable enzyme, such as horseradish peroxidase or alkaline phosphatase), and, together with the unlabeled sample of microvesicles, brought into contact with the corresponding antibody, whereon a second antibody is used to bind the first, and radioactivity or the immobilized enzyme assayed (competitive assay). Alternatively, the protein present on and/or within the microvesicles is allowed to react with the corresponding immobilized antibody, radioisotope or enzyme-labeled anti-marker antibody is allowed to react with the system, and radioactivity or the enzyme assayed (ELISA-sandwich assay). Other conventional methods can also be employed as suitable.

The above techniques can be conducted essentially as a "one-step" or "two-step" assay. A "one-step" assay involves contacting antigen with immobilized antibody and, without washing, contacting the mixture with labeled antibody. A "two-step" assay involves washing before contacting, the mixture with labeled antibody. Other conventional methods can also be employed as suitable.

In certain embodiments, the detection of a protein marker from a donor organ-derived microvesicle sample includes the steps of: contacting the microvesicle sample with an antibody or variant (*e*.*g*., fragment) thereof which selectively binds the protein marker, and detecting whether the antibody or variant thereof is bound to the sample. The method can further include contacting the sample with a second antibody, *e*.*g*., a labeled antibody. The method can further include one or more steps of washing, *e*.*g*., to remove one or more reagents.

It can be desirable to immobilize one component of the assay system on a support, thereby allowing other components of the system to be brought into contact with the component and readily removed without laborious and time-consuming labor. It is possible for a second phase to be immobilized away from the first, but one phase is usually sufficient.

It is possible to immobilize the enzyme itself on a support, but if solid-phase enzyme is required, then this is generally best achieved by binding to antibody and affixing the antibody to a support, models and systems for which are well-known in the art. Simple polyethylene can provide a suitable support.

Enzymes employable for labeling are not particularly limited, but can be selected from the members of the oxidase group, for example. These catalyze production of hydrogen peroxide by reaction with their substrates, and glucose oxidase is often used for its good stability, ease of availability and cheapness, as well as the ready availability of its substrate (glucose). Activity of the oxidase can be assayed by measuring the concentration of hydrogen peroxide formed after reaction of the enzyme-labeled antibody with the substrate under controlled conditions well-known in the art.

Other techniques can be used to detect a protein marker according to a practitioner's preference based upon the present disclosure. One such technique is Western blotting (Towbin et al., Proc. Nat. Acad. Sci. 76:4350 (1979)), wherein a suitably treated sample is run on an SDS-PAGE gel before being transferred to a solid support, such as a nitrocellulose filter. Antibodies (unlabeled) are then brought into contact with the support and assayed by a secondary immunological reagent, such as labeled protein A or anti-immunoglobulin (suitable labels including ¹²⁵I, horseradish peroxidase and alkaline phosphatase). Chromatographic detection can also be used.

Other machine or autoimaging systems can also be used to measure immunostaining results for the marker. As used herein, "quantitative" immunohistochemistry refers to an automated method of scanning and scoring samples that have undergone immunohistochemistry, to identify and quantitate the presence of a specified marker, such as an antigen or other protein. The score given to the sample is a numerical representation of the intensity of the immunohistochemical staining of the sample, and represents the amount of target marker present in the sample. As used herein, Optical Density (OD) is a numerical score that represents intensity of staining. As used herein, semi-quantitative immunohistochemistry refers to scoring of immunohistochemical results by human eye, where a trained operator ranks results numerically (*e*.*g*., as 1, 2 or 3).

Various automated sample processing, scanning and analysis systems suitable for use with immunohistochemistry are available in the art. Such systems can include automated staining (see, *e.g.,* the Benchmark system, Ventana Medical Systems, Inc.) and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples. See, *e.g*., the CAS-200 system (Becton, Dickinson & Co.).

Another method that can be used for detecting protein markers is Western blotting. Immunodetection can be performed with antibody to a protein marker using the enhanced chemiluminescence system (*e*.*g*., from PerkinElmer Life Sciences, Boston, Mass.). The membrane can then be stripped and re-blotted with a control antibody, *e*.*g*., anti-actin (A-2066) polyclonal antibody from Sigma (St. Louis, Mo.).

Antibodies against protein markers can also be used for imaging purposes, for example, to detect the presence of a donor organ-derived microvesicles in a sample of microvesicles obtained from a recipient's blood. Suitable labels include radioisotopes, iodine (¹²⁵I, ¹²¹I), carbon (¹⁴C), sulphur (³⁵S), tritium (³H), indium (¹¹²In), and technetium (^{99m}Tc), fluorescent labels, such as fluorescein and rhodamine and biotin. Immunoenzymatic interactions can be visualized using different enzymes such as peroxidase, alkaline phosphatase, or different chromogens such as DAB, AEC or Fast Red.

For *in vivo* imaging purposes, antibodies are not detectable, as such, from outside the body, and so must be labeled, or otherwise modified, to permit detection. Labels for this purpose can be any that do not substantially interfere with the antibody binding, but which allow external detection. Suitable labels can include those that can be detected by X-radiography, NMR or MRI. For X-radiographic techniques, suitable labels include any radioisotope that emits detectable radiation but that is not overtly harmful to the subject, such as barium or caesium, for example. Suitable labels for NMR and MRI generally include those with a detectable characteristic spin, such as deuterium, which can be incorporated into the antibody by suitable labeling of nutrients for the relevant hybridoma, for example.

The size of the subject, and the imaging system used, will determine the quantity of imaging moiety needed to produce diagnostic images. In the case of a radioisotope moiety, for a human subject, the quantity of radioactivity injected will normally range from about 5 to 20 millicuries of technetium-99 m.

The labeled antibody or antibody fragment will then preferentially accumulate at the location of the sample which contains a protein marker. The labeled antibody or variant thereof, *e*.*g*., antibody fragment, can then be detected using known techniques. Antibodies for use in the present disclosure include any antibody, whether natural or synthetic, full length or a fragment thereof, monoclonal or polyclonal, that binds sufficiently strongly and specifically to the marker to be detected. An antibody can have a K_{d} of at most about 10⁻⁶M, 10⁻⁷M, 10⁻⁸M, 10⁻⁹M, 10⁻¹⁰M, 10⁻¹¹M, 10⁻¹²M. The phrase "specifically binds" refers to binding of, for example, an antibody to an epitope or antigen or antigenic determinant in such a manner that binding can be displaced or competed with a second preparation of identical or similar epitope, antigen or antigenic determinant.

Antibodies and derivatives thereof that can be used encompasses polyclonal or monoclonal antibodies, chimeric, human, humanized, primatized (CDR-grafted), veneered or single-chain antibodies, phase produced antibodies (*e*.*g*., from phage display libraries), as well as functional binding fragments, of antibodies. For example, antibody fragments capable of binding to a marker, or portions thereof, including, but not limited to Fv, Fab, Fab' and F(ab')₂ fragments can be used. Such fragments can be produced by enzymatic cleavage or by recombinant techniques. For example, papain or pepsin cleavage can generate Fab or F(ab')₂ fragments, respectively. Other proteases with the requisite substrate specificity can also be used to generate Fab or F(ab')₂ fragments. Antibodies can also be produced in a variety of truncated forms using antibody genes in which one or more stop codons have been introduced upstream of the natural stop site. For example, a chimeric gene encoding a F(ab')₂ heavy chain portion can be designed to include DNA sequences encoding the CH, domain and hinge region of the heavy chain.

Synthetic and engineered antibodies are described in, *e.g.,* Cabilly et al., U.S. Pat. No. 4,816,567 Cabilly et al., European Patent No. 0,125,023 B1; Boss et al., U.S. Pat. No. 4,816,397; Boss et al., European Patent No. 0,120,694 B1; Neuberger, M. S. et al., WO 86/01533; Neuberger, M. S. et al., European Patent No. 0,194,276 B1; Winter, U.S. Pat. No. 5,225,539; Winter, European Patent No. 0,239,400 B1; Queen et al., European Patent No. 0451216 B1; and Padlan, E. A. et al., EP 0519596 A1. *See* also, Newman, R. et al., BioTechnology, 10: 1455-1460 (1992), regarding primatized antibody, and Ladner et al., U.S. Pat. No. 4,946,778 and Bird, R. E. et al., Science, 242: 423-426 (1988)) regarding single-chain antibodies.

In certain embodiments, agents that specifically bind to a polypeptide other than antibodies are used, such as peptides. Peptides that specifically bind can be identified by any means known in the art, *e.g.,* peptide phage display libraries. Generally, an agent that is capable of detecting a marker polypeptide, such that the presence of a marker is detected and/or quantitated, can be used. As defined herein, an "agent" refers to a substance that is capable of identifying or detecting a protein marker in a sample (*e*.*g*., identifies or detects the mRNA of a marker, the DNA of a marker, the protein of a marker). In certain embodiments, the agent is a labeled or labelable antibody which specifically binds to a marker polypeptide.

In addition, a protein marker can be detected using Mass Spectrometry such as MALDI/TOF (time-of-flight), SELDI/TOF, liquid chromatography-mass spectrometry (LC-MS), gas chromatography-mass spectrometry (GC-MS), high performance liquid chromatography-mass spectrometry (HPLC-MS), capillary electrophoresis-mass spectrometry, nuclear magnetic resonance spectrometry, or tandem mass spectrometry (*e.g*., MS/MS, MS/MS/MS, ESI-MS/MS, etc.). *See,* for example, U.S. Patent Application Nos: 2003/0199001, 2003/0134304, 2003/0077616, which are herein incorporated by reference.

Mass spectrometry methods are well known in the art and have been used to detect biomolecules, such as proteins (*see, e.g*., Li et al. (2000) Tibtech 18:151-160; Rowley et al. (2000) Methods 20: 383-397; and Kuster and Mann (1998) Curr. Opin. Structural Biol. 8: 393-400). Further, mass spectrometric techniques have been developed that permit at least partial de novo sequencing of isolated proteins. Chait et al., Science 262:89-92 (1993); Keough et al., Proc. Natl. Acad. Sci. USA. 96:7131-6 (1999); reviewed in Bergman, EXS 88:133-44 (2000).

In certain embodiments, a gas phase ion spectrophotometer can be used. In other embodiments, laser-desorption/ionization mass spectrometry is used to analyze the sample. Modem laser desorption/ionization mass spectrometry ("LDI-MS") can be practiced in two main variations: matrix assisted laser desorption/ionization ("MALDI") mass spectrometry and surface-enhanced laser desorption/ionization ("SELDI"). In MALDI, the analyte is mixed with a solution containing a matrix, and a drop of the liquid is placed on the surface of a substrate. The matrix solution then co-crystallizes with the biological molecules. The substrate is inserted into the mass spectrometer. Laser energy is directed to the substrate surface where it desorbs and ionizes the biological molecules without significantly fragmenting them. However, MALDI has limitations as an analytical tool. It does not provide means for fractionating the sample, and the matrix material can interfere with detection, especially for low molecular weight analytes. See, *e.g.,* U.S. Pat. No. 5,118,937 (Hillenkamp et al.), and U.S. Pat. No. 5,045,694 (Beavis & Chait).

For additional information regarding mass spectrometers, see, *e*.*g*., Principles of Instrumental Analysis, 3rd edition. Skoog, Saunders College Publishing, Philadelphia, 1985; and Kirk-Othmer Encyclopedia of Chemical Technology, 4th ed. Vol. 15 (John Wiley & Sons, New York 1995), pp. 1071-1094.

Detection of the presence of a marker or other substances can involve detection of signal intensity. This, in turn, can reflect the quantity and character of a polypeptide bound to the substrate. For example, in certain embodiments, the signal strength of peak values from spectra of a first sample and a second sample can be compared (*e*.*g*., visually, by computer analysis etc.), to determine the relative amounts of a particular marker. Software programs such as the Biomarker Wizard program (Ciphergen Biosystems, Inc., Fremont, Calif.) can be used to aid in analyzing mass spectra. The mass spectrometers and their techniques are well known to those of skill in the art.

Any person skilled in the art understands, any of the components of a mass spectrometer (*e*.*g*., desorption source, mass analyzer, detect, etc.) and varied sample preparations can be combined with other suitable components or preparations described herein, or to those known in the art. For example, in some embodiments a control sample can contain heavy atoms (*e.g*., 13C) thereby permitting the test sample to be mixed with the known control sample in the same mass spectrometry run.

In certain embodiments, a laser desorption time-of-flight (TOF) mass spectrometer is used. In laser desorption mass spectrometry, a substrate with a bound marker is introduced into an inlet system. The marker is desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of molecules of specific mass to charge ratio.

### RNA Detection Techniques

In certain embodiments, the methods of the present disclosure can include the detection of one or more markers specific to the donor and/or the donor organ to confirm the proper isolation and/or purification of donor organ-derived exosomes. In certain embodiments, the marker is a nucleic acid, including DNA and/or RNA, contained within the donor organ-specific isolated microvesicles, *e*.*g*., exosomes. Nucleic acid molecules can be isolated from a microvesicle using any number of methods, which are well-known in the art, the particular isolation procedure chosen being appropriate for the particular biological sample. In certain instances, with some techniques, it can also be possible to analyze the nucleic acid without extraction from the microvesicle.

In certain embodiments, the detection of nucleic acids present in the microvesicles can be quantitative and/or qualitative. Any method for qualitatively or quantitatively detecting a nucleic acid marker can be used. Detection of RNA transcripts can be achieved, for example, by Northern blotting, wherein a preparation of RNA is run on a denaturing agarose gel, and transferred to a suitable support, such as activated cellulose, nitrocellulose or glass or nylon membranes. Radiolabeled cDNA or RNA is then hybridized to the preparation, washed and analyzed by autoradiography.

Detection of RNA transcripts can further be accomplished using amplification methods. For example, it is within the scope of the present disclosure to reverse transcribe mRNA into cDNA followed by polymerase chain reaction (RT-PCR); or, to use a single enzyme for both steps as described in U.S. Pat. No. 5,322,770, or reverse transcribe mRNA into cDNA followed by symmetric gap ligase chain reaction (RT-AGLCR) as described by R. L. Marshall, et al., PCR Methods and Applications 4: 80-84 (1994). In certain embodiments, quantitative real-time polymerase chain reaction (qRT-PCR) can be used to detect RNA.

Other known amplification methods which can be utilized herein include, but are not limited to, the so-called "NASBA" or "3SR" technique described in PNAS USA 87: 1874-1878 (1990) and also described in Nature 350 (No. 6313): 91-92 (1991); Q-beta amplification as described in published European Patent Application (EPA) No. 4544610; strand displacement amplification (as described in G. T. Walker et al., Clin. Chem. 42: 9-13 (1996) and European Patent Application No. 684315; and target mediated amplification, as described by PCT Publication WO9322461.

*In situ* hybridization visualization can also be employed. Another method for detecting mRNAs in a microvesicle sample is to detect mRNA levels of a marker by fluorescent *in situ* hybridization (FISH). FISH is a technique that can directly identify a specific sequence of DNA or RNA in a cell, microvesicle sample or biological sample and therefore enables to visual determination of the marker presence and/or expression in tissue samples. Fluorescence *in situ* hybridization is a direct *in situ* technique that is relatively rapid and sensitive. FISH test also can be automated. Immunohistochemistry can be combined with a FISH method when the expression level of the marker is difficult to determine by immunohistochemistry alone.

Alternatively, RNA can be detected on a DNA array, chip or a microarray. Oligonucleotides corresponding to the marker(s) are immobilized on a chip which is then hybridized with labeled nucleic acids of a test sample obtained from a subject. Positive hybridization signal is obtained with the sample containing marker transcripts. Methods of preparing DNA arrays and their use are well known in the art. (See, for example, U.S. Pat. Nos. 6,618,6796; 6,379,897; 6,664,377; 6,451,536; 548,257; U.S. 20030157485 and Schena et al. 1995 Science 20:467-470; Gerhold et al. 1999 Trends in Biochem. Sci. 24, 168-173; and Lennon et al. 2000 Drug discovery Today 5: 59-65, which are herein incorporated by reference in their entirety). Serial Analysis of Gene Expression (SAGE) can also be performed (See for example U.S. Patent Application 20030215858).

To detect RNA molecules, for example, mRNA can be extracted from the microvesicle sample to be tested, reverse transcribed and fluorescent-labeled cDNA probes are generated. The microarrays capable of hybridizing to a marker, cDNA can then probed with the labeled cDNA probes, the slides scanned and fluorescence intensity measured. This intensity correlates with the hybridization intensity and expression levels.

Types of probes for detection of RNA include cDNA, riboprobes, synthetic oligonucleotides and genomic probes. The type of probe used will generally be dictated by the particular situation, such as riboprobes for *in situ* hybridization, and cDNA for Northern blotting, for example. In certain embodiments, the probe is directed to nucleotide regions unique to the particular marker RNA. The probes can be as short as is required to differentially recognize the particular marker RNA transcripts, and can be as short as, for example, 15 bases; however, probes of at least 17 bases, *e.g.,* 18 bases or better 20 bases can be used. In certain embodiments, the primers and probes hybridize specifically under stringent conditions to a nucleic acid fragment having the nucleotide sequence corresponding to the target gene. As herein used, the term "stringent conditions" means hybridization will occur only if there is at least 95% and at least 97% identity between the sequences.

The form of labeling of the probes can be any that is appropriate, such as the use of radioisotopes, for example, ³²P and ³⁵S. Labeling with radioisotopes can be achieved, whether the probe is synthesized chemically or biologically, by the use of suitably labeled bases.

### Kits

In certain non-limiting embodiments, the present disclosure provides for a kit for assessing the conditional state of a transplanted organ in a subject that comprises a means for isolating, purifying and/or detecting one or more donor organ-derived microvesicles.

Types of kits include, but are not limited to, packaged probe and primer sets (e.g., TaqMan probe/primer sets), arrays/microarrays, donor organ and/or donor marker-specific antibodies and antibody-conjugated beads, which further contain one or more probes, primers or other detection reagents for detecting one or more donor organ-derived microvesicles, disclosed herein.

In certain non-limiting embodiments, a kit can comprise a pair of oligonucleotide primers suitable for polymerase chain reaction (PCR) or nucleic acid sequencing, for detecting one or markers of the donor organ-derived microvesicles. A pair of primers can comprise nucleotide sequences complementary to a marker, and be of sufficient length to selectively hybridize with said marker. Alternatively, the complementary nucleotides can selectively hybridize to a specific region in close enough proximity 5' and/or 3' to the marker position to perform PCR and/or sequencing. Multiple marker-specific primers can be included in the kit to simultaneously assay large number of markers. The kit can also comprise one or more polymerases, reverse transcriptase and nucleotide bases, wherein the nucleotide bases can be further detectably labeled.

In certain non-limiting embodiments, a primer can be at least about 10 nucleotides or at least about 15 nucleotides or at least about 20 nucleotides in length and/or up to about 200 nucleotides or up to about 150 nucleotides or up to about 100 nucleotides or up to about 75 nucleotides or up to about 50 nucleotides in length.

In certain non-limiting embodiments, the oligonucleotide primers can be immobilized on a solid surface or support, for example, on a nucleic acid microarray, wherein the position of each oligonucleotide primer bound to the solid surface or support is known and identifiable.

In certain non-limiting embodiments, a kit can comprise at least one nucleic acid probe, suitable for *in situ* hybridization or fluorescent *in situ* hybridization, for detecting the marker and/or the donor organ-derived microvesicles. Such kits will generally comprise one or more oligonucleotide probes that have specificity for various markers.

In certain non-limiting embodiments, a kit can comprise at least one antibody for immunodetection of the marker and/or the donor organ-derived microvesicles and/or for the isolation of the donor organ-derived microvesicles. Antibodies, both polyclonal and monoclonal, specific for a marker, can be prepared using conventional immunization techniques, as will be generally known to those of skill in the art. The immunodetection reagents of the kit can include detectable labels that are associated with, or linked to, the given antibody or antigen itself. Such detectable labels include, for example, chemiluminescent or fluorescent molecules (rhodamine, fluorescein, green fluorescent protein, luciferase, Cy3, Cy5, or ROX), radiolabels (3H, 35S, 32P, 14C, 1311) or enzymes (alkaline phosphatase, horseradish peroxidase).

In certain non-limiting embodiments, the antibody can be provided bound to a solid support, such as a column matrix, an array, or well of a microtiter plate. Alternatively, the support can be provided as a separate element of the kit.

In certain non-limiting embodiments, a kit can comprise one or more primers, probes, microarrays, or antibodies suitable for detecting one or more markers.

In certain non-limiting embodiments, where the measurement means in the kit employs an array, the set of markers set forth above can constitute at least 10 percent or at least 20 percent or at least 30 percent or at least 40 percent or at least 50 percent or at least 60 percent or at least 70 percent or at least 80 percent of the species of markers represented on the microarray.

In certain non-limiting embodiments, a marker detection kit can comprise one or more detection reagents and other components (e.g., a buffer, enzymes such as DNA polymerases or ligases, chain extension nucleotides such as deoxynucleotide triphosphates, and in the case of Sanger-type DNA sequencing reactions, chain terminating nucleotides, positive control sequences, negative control sequences, and the like) necessary to carry out an assay or reaction to detect a marker. A kit can also include additional components or reagents necessary for the detection of a marker, such as secondary antibodies for use in immunohistochemistry. A kit can further include one or more other markers or reagents for evaluating other prognostic factors, *e.g.,* stage of rejection.

In certain non-limiting embodiments, a marker detection kit can comprise one or more reagents and/or tools for isolating donor organ-specific microvesicles from a biological sample. A kit can also include reagents necessary for isolating the protein and/or nucleic acids from the isolated microvesicles.

### Reports, Programmed Computers, and Systems

In certain embodiments, the monitoring of the conditional state of a transplanted organ in a subject based on the isolation, purification and/or detection of donor organ-derived microvesicles, can be referred to herein as a "report". A tangible report can optionally be generated as part of a testing process (which can be interchangeably referred to herein as "reporting," or as "providing" a report, "producing" a report, or "generating" a report).

Examples of tangible reports can include, but are not limited to, reports in paper (such as computer-generated printouts of test results) or equivalent formats and reports stored on computer readable medium (such as a CD, USB flash drive or other removable storage device, computer hard drive, or computer network server, etc.). Reports, particularly those stored on computer readable medium, can be part of a database, which can optionally be accessible via the internet (such as a database of patient records or genetic information stored on a computer network server, which can be a "secure database" that has security features that limit access to the report, such as to allow only the patient and the patient's medical practitioners to view the report while preventing other unauthorized individuals from viewing the report, for example). In addition to, or as an alternative to, generating a tangible report, reports can also be displayed on a computer screen (or the display of another electronic device or instrument).

A report can include, for example, an individual's medical history, or can just include size, presence, absence or levels of one or more markers (for example, a report on computer readable medium such as a network server can include hyperlink(s) to one or more journal publications or websites that describe the medical/biological implications). Thus, for example, the report can include information of medical/biological significance as well as optionally also including information regarding the detection of organ donor-derived microvesicles, or the report can just include information regarding the detection of organ donor-derived microvesicles without other medical/biological significance.

A report can further be "transmitted" or "communicated" (these terms can be used herein interchangeably), such as to the individual who was tested, a medical practitioner (e.g., a doctor, nurse, clinical laboratory practitioner, genetic counselor, etc.), a healthcare organization, a clinical laboratory, and/or any other party or requester intended to view or possess the report. The act of "transmitting" or "communicating" a report can be by any means known in the art, based on the format of the report. Furthermore, "transmitting" or "communicating" a report can include delivering a report ("pushing") and/or retrieving ("pulling") a report. For example, reports can be transmitted/communicated by various means, including being physically transferred between parties (such as for reports in paper format) such as by being physically delivered from one party to another, or by being transmitted electronically or in signal form (e.g., via e-mail or over the internet, by facsimile, and/or by any wired or wireless communication methods known in the art) such as by being retrieved from a database stored on a computer network server, etc.

In certain exemplary embodiments, the disclosed subject matter provides computers (or other apparatus/devices such as biomedical devices or laboratory instrumentation) programmed to carry out the methods described herein. In certain embodiments, the system can be controlled by the individual and/or their medical practitioner in that the individual and/or their medical practitioner requests the test, receives the test results back, and (optionally) acts on the test results to reduce the individual's disease risk, such as by implementing a disease management system.

The following examples are offered to more fully illustrate the disclosure, but are not to be construed as limiting the scope thereof.

### EXAMPLE 1: Detection of Donor Organ Specific Microvesicles in Recipient's Bloodstream.

There is a significant microvesicle contribution into the blood from immune cells, platelets, and endothelial cells, which results in a large noise-to-signal ratio when a general microvesicle profiling approach is performed. Similar problem exists with studies analyzing circulating free protein and nucleic acid markers. In addition, free protein and nucleic acid markers are unstable in the circulation, thus requiring a high steady-state for detection, and minor changes over time (essential for monitoring) are difficult to quantify. However, microvesicles and their RNA cargo are extremely stable in the circulation. Therefore, transplant organ-specific microvesicles were isolated from the recipient's bloodstream to define states of rejection and/or injury of a transplanted organ.

Donor exosomes released from the transplanted organ were isolated in a xenogeneic islet transplant model, where human islets were transplanted into a nude mouse (n=8). Normoglycemic recipients were sacrificed at various time points (range 31 to 198 days), and the donor human islet mass was stained for insulin for histologic confirmation. Exosomes from recipient plasma was analyzed on a NanoSight NS300 with nanoparticle tracking analysis (NTA) upon fluorescent quantum dot labeling with anti-human specific CD63 (exosome marker) antibody. In all 8 recipient animals, anti-human CD63 signal was observed (absent in negative controls) (p=0.006) (Figure 1A, B). Western blot analysis confirmed detection of human CD63 protein (Figure 1C). Similar findings were confirmed using anti-HLA-C specific antibody. This concept was also tested in the allogeneic setting of tolerance (BALB/c islets into diabetic B6 mouse, n=3), with recipients maintaining normoglycemia >300 days. Recipient plasma exosomes showed donor exosome signal (anti-H2-K^{d} BALB/c specific antibody-quantum dot) signal by NanoSight, which was confirmed by Western blot analysis (absent in negative controls).

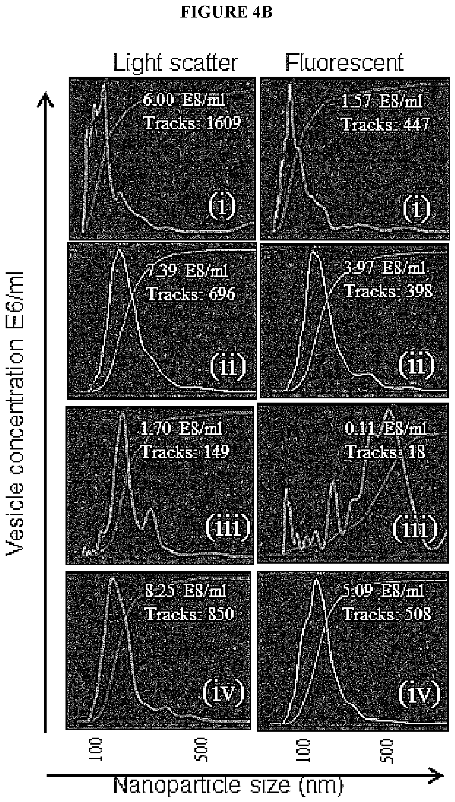

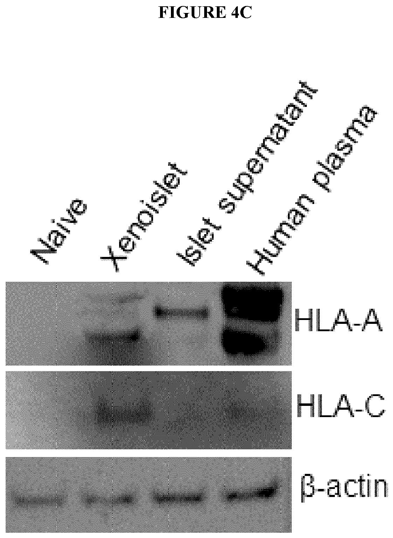

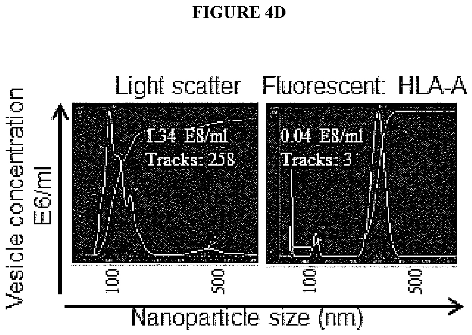

In further experiments, exosomes were isolated from recipient mouse plasma and analyzed for donor islet specific exosomes signal on the NanoSight NS300 nanoparticle detector using anti-human MHC specific antibody-quantum dot. At all tested post-transplant time points, HLA specific exosome signal was detected on NanoSight fluorescence (range 14 to 198 days, n=20) (Figure 4A, B). This signal was absent in naive mouse (n=5) and in allogeneic mouse islet transplant (n=5) plasma exosome samples (p<0.0001). Western blot analysis showed HLA presence in xenoislet samples (Figure 4C). To validate transplant exosome signal specificity, islet graftectomy was performed in xenoislet animals and this led to loss of the HLA-A signal (n=6, p<0.001) (Figure 4D).

To determine if this concept is applicable with other organ transplants, allogeneic full mismatch heterotopic heart transplants were performed in the mouse, where a BALB/c heart was transplanted into B6 recipient (n=10). In this acute rejection model, recipient animals were sacrificed as early as 4 hours post-transplant to 11 days. Plasma exosome pool was isolated from recipient blood using Sepharose gel filtration chromatography, and was assessed at different post-transplant time points for donor heart-specific exosome signal utilizing NanoSight nanoparticle tracking analysis technology with quantum dot labeled anti-donor MHC specific antibodies. At all time points, donor heart-specific exosome signal (anti-H2-K^{d} BALB/c specific quantum dot) was observed by NanoSight analysis, which was confirmed by Western blot (p=0.003). Western blot was also performed for confirmation. Syngeneic B6 transplants served as negative control (n=3), and the signal was absent in the control animals (Figure 2).

These studies in mouse models established that transplanted organ/tissue releases a detectable and stable donor tissue specific exosome pool into recipient blood that can be serially tracked over long term follow-up. Donor specific exosomes were detectable in the human clinical setting as well.

### EXAMPLE 2: Characterization of Donor-Derived Microvesicles Isolated From Recipient Patient's Blood and Urine Samples in the Clinical Setting of Human Living Donor Renal Transplantation.

To test if transplant tissue specific exosome platform can be translated to other transplant tissues and bodily fluids, donor kidney specific exosomes in recipient patient plasma and urine in the setting of human living donor renal transplantation were isolated.

For analysis of microvesicles in the plasma of a donor kidney recipient, 0.5-2 ml of plasma was obtained through venipuncture and stored at -80 °C. For analysis of microvesicles in the urine of a donor kidney recipient, urine samples (40 ml) were collected in sterile cups, treated with 1x protease inhibitor cocktail (Sigma-Aldrich, Co., St. Louis, MO) and frozen at -80 °C until analysis. Exosome isolation from human plasma samples was performed utilizing 500 µl to 1 ml plasma obtained after centrifugation of the blood sample at 500 g for 10 minutes. The plasma sample was directly added to a Sepharose 2B column and the eluent was collected in 1 ml fractions. The exosome fraction was pooled after monitoring absorbance at 280 nm. The pooled fraction was ultracentrifuged at 110,000 g for 2 hours at 4 °C, and the pelleted exosome fraction was resuspended in PBS for downstream analysis. Urinary microvesicle isolation was performed as described elsewhere with slight modification (Rood et al. (2010), Pisitkun et al. (2004)). Briefly, urinary cell debris was removed from 20 ml starting material by centrifugation at 17,000 g for 15 minutes. The supernatant was then ultracentrifuged at 200,000 g for 120 minutes at 4 °C. The pellet was resuspended in PBS and loaded onto a Sepharose 2B size exclusion column, and the eluted fractions representing exosomes were pooled. The pooled fractions were concentrated on an Amicon filter (Merck Millipore Ltd., Ireland) with 100 kDa cut-off membrane. The isolated microvesicle pool were then analyzed on the NanoSight NS300.

The isolated microvesicle pool was utilized for affinity separation of donor kidney specific microvesicle subset from the recipient plasma. Similar affinity separation of donor microvesicle subset was performed with the urine samples. HLA cross-matching of all living donor renal transplants provided a panel of anti-donor specific HLA antibodies to utilize for affinity isolation of donor microvesicle pool. Class I (HLA-A, B, or C) mismatch that gave the strongest signal on HLA cross-matching (serotyping antibody binds to donor cell but not recipient cell) was focused on. First, anti-donor HLA antibody on total microvesicle pools from samples using the light and fluorescent (anti-donor HLA antibody-quantum dot) scatter modes on the NanoSight NS300 was tested. This confirmed which antibody can be utilized for affinity based magnetic bead isolation of donor microvesicle pool. The microvesicle subset was then analyzed on NanoSight NS300 NTA on the light scatter mode to quantitate the donor specific microvesicle signal, and on the fluorescent mode (anti-donor HLA antibody-quantum dot) to confirm enrichment of the donor microvesicle population. The unbound fraction from affinity isolation, which represents the recipient microvesicle pool, was also analyzed to confirm the absence of the donor HLA signal on the fluorescent mode.

Microvesicles in urine and plasma were isolated strictly based on the HLA mismatch and the donor specific microvesicles subset was analyzed for organ specific proteins, *e*.*g*., renal epithelial cell protein, Aquaporin 2. In addition, anti-donor HLA antibody was utilized to confirm enrichment of the donor specific microvesicle subset, and anti-recipient HLA specific antibody confirmed the absence of the recipient microvesicle in the affinity isolated donor microvesicle subset. Unconjugated HLA allele specific antibodies (mouse anti-HLA-A2, -HLA-B27, -HLA-B13, -HLA-B8) were purchased from One Lambda (CA, USA), for donor HLA class I donor type specific exosome isolation and analysis from recipient plasma and urine.

As shown in Figure 3A, the donor was HLA-A2, HLA-B27 positive and the recipient was HLA-A29, HLA-31, HLA-B31 and HLA-B44 positive. Therefore, anti-donor specific antibodies, HLA-A2 and HLA-B27, were used for detection and purification of donor specific exosomes from recipient plasma. Donor specific exosomes were purified through the use of antibody-conjugated magnetic beads. In brief, a MHC specific antibody was covalently conjugated to N-hydroxysuccinamide magnetic beads (Pierce) per manufacturer's protocol. 50 to 100 µg protein equivalent of exosomes were incubated with antibody beads overnight at 4°C. The bead bound and unbound exosomes fractions were separated per manufacturer's protocol. Exosomes bound to beads were eluted using tris glycine and utilized for downstream analysis.

As shown in Figure 3B, HLA-A2 and HLA-B27 positive donor kidney specific exosomes were present in recipient patient plasma and in the donor plasma sample as analyzed by NanoSight. Purified exosomes were analyzed on the NanoSight NS300 (405 nm laser diode) on the light scatter mode for exosome quantification and scatter distribution according to manufacturer's protocols (Malvern instruments Inc., MA, USA). Before each experimental run, the machine was calibrated for nanoparticle size and quantity using standardized nanoparticle and dilutions provided by the manufacturer. Surface marker detection on exosomes was performed using the fluorescence mode on the NanoSight NS300. Secondary antibodies conjugated to quantum dots with emission at 605 nm were utilized for fluorescence detection of primary antibodies binding against specific surface proteins (HLA-A, B, C, B27, A2, B13; FXYD2; CD3, CD4, CD8, CD14, CD56, CD19, mouse MHC I) as described previously (Gardiner et al. (2013), Dragovic et al. (2011)). Each experimental run was performed in duplicates, and an appropriate IgG isotype control fluorescence was performed to assess background.

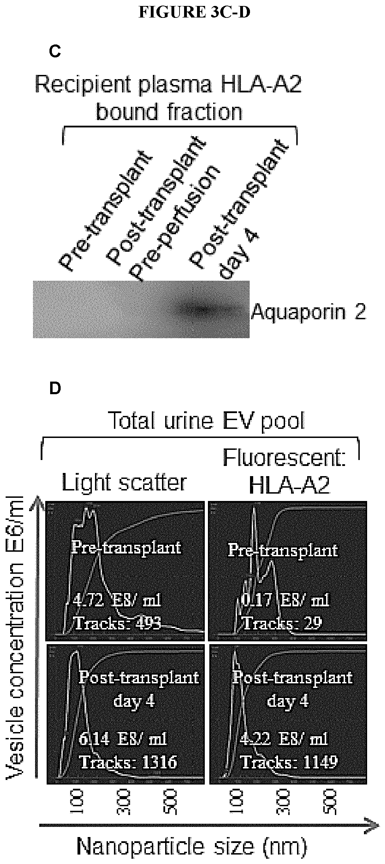

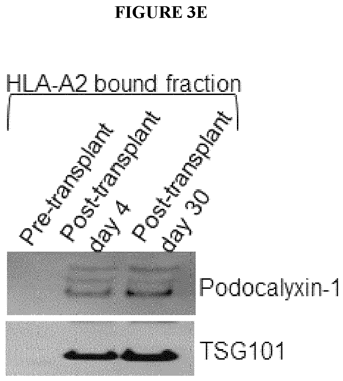

To determine if the exosomes were derived from the proper organ, tissue-specific proteins present on or within the exosomes were analyzed by Western Blot. Western Blot analysis was performed as follows: exosomes and cell lysate total proteins were isolated by pelleting the exosomes and lysing the pellet in 1X RIPA buffer with 1X concentration of protease inhibitor cocktail (Sigma-Aldrich Co., MO). The isolated proteins were separated on polyacrylamide gels and transferred on polyvinylidene difluoride membrane (Life Technologies, NY, USA). The membrane was blocked, incubated with the desired antibody at a concentration per the manufacturer's protocol. Horseradish peroxidase coupled secondary antibody (Santa Cruz Biotechnologies Inc.) was added per manufacturer's protocol and detected through chemiluminescence using Image quant LAS 400 Phospho-Imager (GE Health, USA). Expression of the renal epithelial cell protein, Aquaporin 2, on the donor kidney specific exosome was observed by Western Blot by day 4 post-transplant (Figure 3C). HLA-A2 bound plasma exosomes fractions from recipient pre-transplant and post-kidney implantation but pre-perfusion of the donor organ were negative for aquaporin 2 (Figure 3C). These findings were also validated in recipient urine exosomes (Figure 3D, E). As shown in Figure 3D, a HLA-A2 signal was absent in the pre-transplant sample but the majority of exosomes from the post-transplant day 4 sample were positive for HLA-A2. By Western Blot, HLA-A2 bound urinary exosomes fractions from postoperative day 4 and day 30 showed expression of renal glomerular marker, podocalyxin-1, but the exosomes from the pre-transplant sample did not (Figure 3E).

The following clauses, which are not claims, reflect the original as-filed PCT claims and are intended to ensure that this application includes the full disclosure of the parent PCT application, and represent aspects of the invention.
Clause 1. A method for monitoring transplanted organ status in a subject, comprising:
   (a) obtaining a biological sample from a subject; and
   (b) isolating, purifying or identifying a donor organ-derived microvesicle from the biological sample.
Clause 2. The method of clause 1, wherein the subject is human.
Clause 3. The method of clause 1, wherein the biological sample is selected from the group consisting of a blood sample or a urine sample.
Clause 4. The method of clause 1, wherein the donor organ is selected from the group consisting of a heart, kidney and pancreatic islet.
Clause 5. A method for isolating, purifying or identifying donor-derived microvesicles, comprising:
   (a) obtaining a biological sample from the subject; and
   (b) isolating, purifying or identifying a donor organ-derived microvesicle from the biological sample by detecting a marker specific for the donor.
Clause 6. The method of clause 5, wherein the marker is a protein.
Clause 7. The method of clause 6, wherein the protein is a surface protein.
Clause 8. The method of clause 6, wherein the protein is a major histocompatibility complex protein.
Clause 9. A method for isolating, purifying or identifying donor-derived microvesicles, comprising:
   (a) obtaining a biological sample from the subject; and
   (b) isolating, purifying or identifying a donor organ-derived microvesicle from the biological sample by detecting a marker specific for a cell type present within the donor organ.
Clause 10. The method of clause 5 or 9, wherein the marker is Aquaporin 2.
Clause 11. The method of clause 5 or 9, wherein the donor organ is selected from the group consisting of a heart, kidney and pancreatic islet.
Clause 12. A kit for monitoring transplanted organ status in a subject, comprising reagents useful for detecting a marker specific to a donor organ-derived microvesicle.
Clause 13. The kit of clause 12, comprising a packaged probe and primer set, arrays/microarrays, marker-specific antibodies or marker-specific antibody-conjugated beads.
Clause 14. The kit of clause 12, comprising a pair of oligonucleotide primers, suitable for polymerase chain reaction or nucleic acid sequencing, for detecting the marker.
Clause 15. The kit of clause 12, comprising a monoclonal antibody or antigen-binding fragment thereof, or a polyclonal antibody or antigen-binding fragment thereof, for detecting the marker.

### REFERENCES

Al-Nedawi, K., B. Meehan, J. Micallef, V. Lhotak, L. May, A. Guha, and J. Rak. 2008. Intercellular transfer of the oncogenic receptor EGFRvIII by microvesicles derived from tumour cells. Nat Cell Biol. 10:619-24.
Aquino-Dias, E.C. et al. Non-invasive diagnosis of acute rejection in kidney transplants with delayed graft function. Kidney Int. 73, 877-884 (2008).
Balzar, M., M. J. Winter, C. J. de Boer, and S. V. Litvinov. 1999. The biology of the 17-1A antigen (Ep-CAM). J Mol Med. 77:699-712.
Bossi, A., F. Bonini, A. P. Turner, and S. A. Piletsky. 2007. Molecularly imprinted polymers for the recognition of proteins: the state of the art. Biosens Bioelectron. 22:1131-7.
Cheruvanky, A., H. Thou, T. Pisitkun, J. B. Kopp, M. A. Knepper, P. S. Yuen, and R. A. Star. 2007. Rapid isolation of urinary exosomal biomarkers using a nanomembrane ultrafiltration concentrator. Am J Physiol Renal Physiol. 292:F1657-61.
Delcayre A., Shu H., Le Pecq J. B. Dendritic cell-derived Exosomes in Cancer Immunotherapy: Exploiting Nature's Antigen Delivery Pathway. Expert Rev. AntiCancer Therapy 5(3), in press (2005).
Dragovic, R.A. et al. Sizing and phenotyping of cellular vesicles using Nanoparticle Tracking Analysis. Nanomedicine. 7(6), 780-788 (2011).
Escudier B, Dorval T, Chaput N et al. Vaccination of metastatic melanoma patients with autologous dendritic cell (DC) derived exosomes: results of the first phase I clinical trial. J. Transl. Med. 3(1), 10 (2005).
Gardiner, C., Ferreira, Y.J., Dragovic, R.A., Redman, C.W. and Sargent, I.L. Extracellular vesicle sizing and enumeration by nanoparticle tracking analysis. J Extracell Vesicles. 2, 19671 (2013).
Johnson, S., D. Evans, S. Laurenson, D. Paul, A. G. Davies, P. K. Ferrigno, and C. Walti. 2008. Surface-immobilized peptide aptamers as probe molecules for protein detection. Anal Chem. 80:978-83.
Keller, S., C. Rupp, A. Stoeck, S. Runz, M. Fogel, S. Lugert, H. D. Hager, M. S. Abdel-Bakky, P. Gutwein, and P. Altevogt. 2007. CD24 is a marker of exosomes secreted into urine and amniotic fluid. Kidney Int. 72:1095-102.
Kotsch, K. et al. Enhanced granulysin mRNA expression in urinary sediment in early and delayed acute renal allograft rejection. Transplantation. 77, 1866-1875 (2004).
Li, B. et al. Noninvasive diagnosis of renal-allograft rejection by measurement of messenger RNA for perforin and granzyme B in urine. N Engl J Med. 344, 947-954 (2001).
Morse M A, Garst J, Osada T, et al. A phase I study of dexosome immunotherapy in patients with advanced non-small cell lung cancer. J. Transl. Med. 3(1), 9 (2005).
Pisitkun, T., Shen, R-F. & Knepper, M.A. Identification and proteomic profiling of exosomes in human urine. Proc Natl Acad Sci USA. 101(36), 13368-13373 (2004).
Raposo, G., H. W. Nijman, W. Stoorvogel, R. Liejendekker, C. V. Harding, C. J. Melief, and H. J. Geuze. 1996. B lymphocytes secrete antigen-presenting vesicles. J Exp Med. 183: 1161-72.
Rood, I.M. et al. Comparison of three methods for isolation of urinary microvesicles to identify biomarkers of nephrotic syndrome. Kidney Int. 78(8), 810-6 (2010).
Suthanthiran, M. et al. Urinary-cell mRNA profile and acute cellular rejection in kidney allografts. N Engl J Med. 369, 20-31 (2013).
Tatapudi, R.R. et al. Noninvasive detection of renal allograft inflammation by measurements of mRNA for IP-10 and CXCR3 in urine. Kidney Int. 65, 2390-2397 (2004).
Taylor, D. D., and C. Gercel-Taylor. 2008. MicroRNA signatures of tumor-derived exosomes as diagnostic biomarkers of ovarian cancer. Gynecol Oncol. 110:13-21.
Taylor, D.D., Gercel-Taylor, C. Tumor-derived exosomes as mediates of T-cell signaling defects. British Journal of Cancer. 2005. 92: 305-311.
Taylor, D.D., Zacharias, W., and Gercel-Taylor, C. Exosome isolation for proteomic analyses and RNA profiling. In: Methods in Molecular Biology (RJ Simpson, ed). 2011. 728: 235-246.
Thery C, Zitvogel Z, Amigorena S. Exosomes: composition, biogenesis and function. Nat. Rev. Immunol. 2(8), 569-579 (2002).
Zitvogel L, Regnault A, Lozier A et al. Eradication of established murine tumors using a novel cell-free vaccine: dendritic cell-derived exosomes. Nat. Med. 4(5), 594-600 (1998).

Various publications, patents and patent applications are cited herein, the contents of which are hereby incorporated by reference herein in their entireties.

## Claims

1. A kit for monitoring transplanted organ status in a subject, comprising reagents for detecting a major histocompatibility complex marker specific to a donor organ-derived microvesicle.

2. The kit of claim 1, comprising a packaged probe and primer set, arrays/microarrays, marker-specific antibodies or marker-specific antibody-conjugated beads.

3. The kit of claim 1, comprising a pair of oligonucleotide primers, suitable for polymerase chain reaction or nucleic acid sequencing, for detecting the marker.

4. The kit of claim 1, comprising a monoclonal antibody or antigen-binding fragment thereof, or a polyclonal antibody or antigen-binding fragment thereof, for detecting the marker.

5. A method of making a kit for monitoring transplanted organ status in a subject, the method comprising creating a reagent for the kit based on a major histocompatibility complex marker specific to a donor organ-derived microvesicle; and keeping the reagent as part of the kit.

6. Use of the kit of claim 1 or a kit produced by the method of claim 5 to monitor a transplanted organ status in a subject.
